# FASCICULE DE BREVET EUROPEEN

(11) **EP 1 865 828 B1**
(45) Date de publication et mention de la délivrance du brevet: **03.05.2017**
(21) Numéro de dépôt: 06726231.1
(22) Date de dépôt: 10.03.2006
(51) Int. Cl.: A61B 3/113

(54) **PROCÉDÉ ET DISPOSITIF DE DÉTERMINATION DU CENTRE DE ROTATION D'UN OEIL**
VERFAHREN UND VORRICHTUNG ZUR BESTIMMUNG DES ROTATIONSZENTRUMS DES AUGES
METHOD AND DEVICE FOR DETERMINING THE EYE'S ROTATION CENTER

(30) Priorité: 08.04.2005 FR 0550902
(43) Date de publication de la demande: 19.12.2007
(73) Titulaire: ESSILOR INTERNATIONAL (Compagnie Générale d'Optique), 94220 Charenton-le-Pont (FR)
(72) Inventeur: BONNIN, Thierry, F-94227 Charenton-le-pont (FR); DECRETON, Bruno, F-94227 Charenton-le-pont (FR); MARIN, Gildas, F-94227 Charenton-le-pont (FR); PETIGNAUD, Cécile, F-94227 Charenton-le-pont (FR)
(74) Mandataire: Lenne, Laurence
(86) Numéro de dépôt international: PCT/FR2006/050207
(87) Numéro de publication internationale: WO 2006/106248

(56) Documents cités:
- US-A1- 2003 169 907
- US-B1- 6 580 448
- FRY G A; HILL W W: "The center of rotation of the eye." AMERICAN JOURNAL OF OPTOMETRY AND ARCHIVES OF AMERICAN ACADEMY OF OPTOMETRY, vol. 39, novembre 1962 (1962-11), pages 581-595, XP009055840 US ISSN: 0002-9408

## Description

L'invention concerne un procédé et un dispositif de détermination du centre de rotation d'un oeil.

L'invention est destinée de préférence, mais non limitativement, à une application ophtalmique.

Lorsqu'on s'intéresse à la vision d'une personne, on cherche à qualifier la vision dans tout le champ de vision, en regard droit devant et lorsque l'oeil fixe différents points de l'espace. Pour cela, il est nécessaire de prendre en compte le mouvement des yeux de cette personne lorsqu'elle fixe différents points du champ. Le mouvement de l'oeil est couramment assimilé à une rotation autour d'un point particulier, appelé le CRO (Centre de Rotation de l'oeil).

Les caractéristiques optiques d'un système verre-oeil dépendent de la position du CRO par rapport aux lunettes et on considère habituellement que le CRO est placé sur la direction primaire de regard. De manière habituelle, on peut mesurer, par simple photographie, la distance qui sépare la face arrière du verre, de la face avant de la cornée suivant la direction primaire de regard. Le CRO est ensuite positionné à une distance paramétrée, qui peut être égale à 15 mm en arrière de la face avant de la cornée.

De façon plus générale, la mesure de la position du CRO est faite par rapport à un point de la face arrière ou de la face avant du verre de lunettes, ou un point particulier du visage, par rapport auquel on saura également positionner le verre.

Ces valeurs habituellement utilisées pour déterminer le CRO sont des valeurs théoriques, arbitraires et standards.

Or, dans la pratique, l'oeil a une position et des dimensions différentes selon les personnes et de ce fait, le positionnement du CRO par cette valeur théorique est approximatif. D'une façon plus générale, les mouvements de l'oeil et donc la position du CRO peuvent être caractérisés par une fonction dépendant de la direction de regard.

L'invention a pour objet de déterminer la position du CRO par mesure personnalisée sur la personne.

Dans le domaine ophtalmique, cette détermination personnalisée permet la mise au point de verres ophtalmiques réalisés sur mesure et plus performants.

Dans un domaine d'application plus général, l'invention permet une détermination précise du CRO. Celle-ci est notamment nécessaire dans des applications de simulation en réalité virtuelle où les caméras doivent être positionnées précisément sur les CRO pour un rendu stéréo 3D correct.

Il est connu de façon théorique de déterminer le centre de rotation d'un oeil d'une personne par rapport à un repère lié à la personne, par détermination de l'axe visuel de cette personne dans au moins deux directions non parallèles au moyen d'une cible visualisée et par définition d'un point optimal dit de croisement de ces axes comme centre de rotation de l'oeil.

Cependant ce procédé pose le problème technique suivant.

Pour une définition précise, il est nécessaire d'immobiliser la tête de la personne au moyen d'un dispositif spécifique du type mentonnière. En effet, de par un mouvement réflexe, lors du déplacement de l'oeil, même sur un angle relativement petit, la personne a tendance à bouger la tête et ce déplacement entraîne une définition systématiquement approximative.

Un dispositif pour la détermination du centre de rotation d'un oeil d'une personne par rapport à un repère lié à la personne, permettant la détermination de l'axe visuel de cette personne dans au moins deux directions non parallèles au moyen d'une cible visualisée et la définition d'un point optimal dit de croisement de oes axes comme centre de rotation de l'oeil, au moins deux positions relatives de cette cible et de la tête de la personne étant mesurées, est décrit dans le document de brevet US 6 580 448.

Cependant, le dispositif proposé dans ce document est particulièrement complexe quant à sa constitution et à sa manipulation.

L'invention propose un dispositif spécifiquement destiné à la détermination du centre de rotation d'un oeil d'une personne qui est de constitution très simple et d'une grande facilité de manipulation.

L'invention résout ce problème et pour ce faire elle propose un procédé de détermination du centre de rotation d'un oeil d'une personne par rapport à un repère lié à la personne ou à sa paire de lunettes, consistant en la détermination de l'axe visuel de cette personne dans au moins deux directions non parallèles au moyen d'une cible visualisée et la définition d'un point optimal dit de croisement de ces axes comme centre de rotation de l'oeil, caractérisé en ce qu' au moins deux positions relatives de cette cible et de la tête de la personne sont mesurées, et en ce qu'il consiste, pour chacune de ces directions non parallèles, à saisir, positionner et bouger ladite cible constituée d'une source de lumière ponctuelle disposée à une extrémité d'un support tubulaire constitué uniquement d'un tube et portant un premier capteur de position, calibré pour connaître la direction du faisceau lumineux de ladite lumière ponctuelle, avec son autre extrémité en face de l'oeil de la personne, de façon à superposer l'axe visuel avec chaque direction du faisceau lumineux de la source de lumière, un second capteur de position étant placé sur la tête de la personne et un logiciel associé aux deux dits capteurs assurant l'enregistrement de la direction de l'axe visuel, ainsi que la position de la tête correspondante, puis le calcul de l'axe visuel dans un repère lié à la tête.

Grâce à l'invention, la personne peut être dans une position naturelle ou mobile.

Dans une première variante de réalisation, ladite cible est une source de lumière ponctuelle et unidirectionnelle, de préférence un laser, qui assure que le support est aligné avec la direction de regard dès que la personne voit la cible.

Dans une deuxième variante de réalisation, ladite cible est une source de lumière ponctuelle quelconque, de préférence une diode ou une mire, et le support comporte une seconde cible transparente, constituée d'une lame transparente réticulée ou d'un écran percé d'un trou, destinée à être disposée en face de l'oeil de la personne et qui assure que le support est aligné avec la direction de regard dès que la personne voit les deux cibles alignées.

Selon une troisième variante, ledit support est un tube de faible diamètre qui assure l'alignement avec la direction de regard dès que la personne voit la cible.

Avantageusement, ces trois variantes peuvent être combinées pour améliorer la précision de l'alignement et/ou faciliter le calibrage de l'appareil.

Avantageusement, une autre cible transparente, constituée d'une lame transparente réticulée ou d'un écran percé d'un trou, peut être disposée entre la première cible et le milieu du support quand celle-ci est une source lumineuse, de façon à améliorer la précision de l'alignement en précisant la position de la cible et à faciliter le calibrage de l'appareil.

Le dispositif conforme à l'invention peut comprendre un écran fixe par rapport à la tête de la personne et disposé devant l'oeil de la personne et pourvu de zones ponctuelles translucides correspondantes aux dites directions.

Cet écran peut être fixé sur une monture de lunette ou un casque.

Avantageusement, les zones ponctuelles translucides, qui peuvent être de simples trous, ont un diamètre plus petit que le diamètre naturel de la pupille de l'oeil de la personne, de préférence compris entre 0,5 et 2 mm.

Selon une variante de réalisation, la définition du point optimal de croisement consiste en la définition d'une surface perpendiculaire à chacun desdits axes visuels et en la définition du meilleur foyer de cette surface, ce meilleur foyer étant défini comme le centre de rotation de l'oeil.

Selon une autre variante de réalisation, la définition du point optimal de croisement consiste en la définition, pour chaque couple d'axes visuels, du point équidistant à ces deux axes et de distance minimale à ces deux axes, en la définition du barycentre de ces dits points, ce barycentre étant défini comme le centre de rotation de l'oeil.

Selon une troisième variante de réalisation, la définition du point optimal de croisement consiste en la définition, pour chaque couple d'axes visuels, du point équidistant à ces deux axes et de distance minimale à ces deux axes, en la définition d'une sphère de rayon minimal contenant lesdits points, le centre de cette sphère étant défini comme le centre de rotation de l'oeil.

Le procédé est optimisé et permet une précision de détermination du CRO inférieure au millimètre.

Avantageusement, lesdites directions repérées sont sensiblement symétriques par rapport au regard droit devant de l'oeil.

L'invention est décrite plus en détail ci-dessous à l'aide de figures ne représentant qu'un mode de réalisation préféré de l'invention
La figure 1 est une vue schématique d'un dispositif conforme à l'invention, selon une première variante de réalisation.
La figure 2 est une vue schématique d'un dispositif conforme à l'invention, selon une seconde variante de réalisation.
La figure 3 est une vue du dispositif conforme à l'invention porté par une personne.

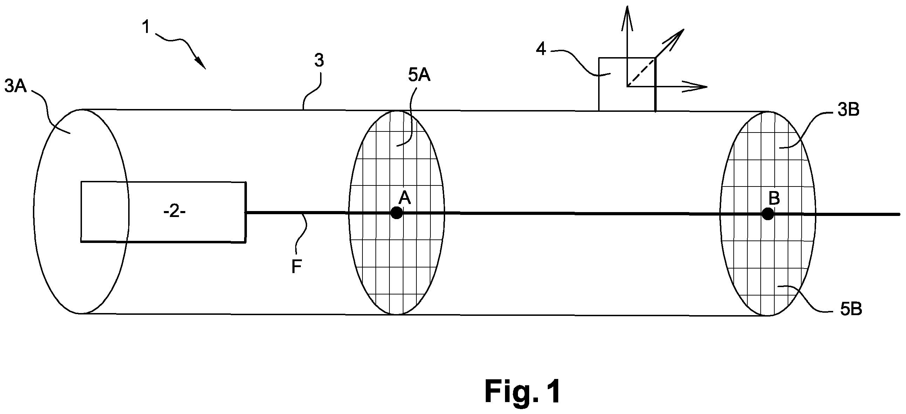

Comme représenté sur la figure 1, un dispositif 1 conforme à l'invention comporte une cible, de préférence constituée d'une source lumineuse 2, disposée à l'extrémité 3A d'un support 3, par exemple formé d'un conduit cylindrique ou tube, dont l'autre extrémité 3B est destinée à être disposée en face de l'oeil de la personne.

Deux lames transparentes réticulée 5A, 5B sont disposées dans le tube, de préférence, l'une 5A est disposée à proximité de la source lumineuse 2, par exemple au milieu longitudinal du tube, et l'autre 5B est disposée à l'extrémité ouverte 3B du tube. Ces lames peuvent être remplacées par des écrans percés d'un trou central.

La lame 5B disposée à l'extrémité ouverte 3B du tube est optionnelle si la source lumineuse 2 est ponctuelle et unidirectionnelle, tel que par exemple un laser. La lame 5A disposée à proximité de la source lumineuse 2 est optionnelle dans tous les cas.

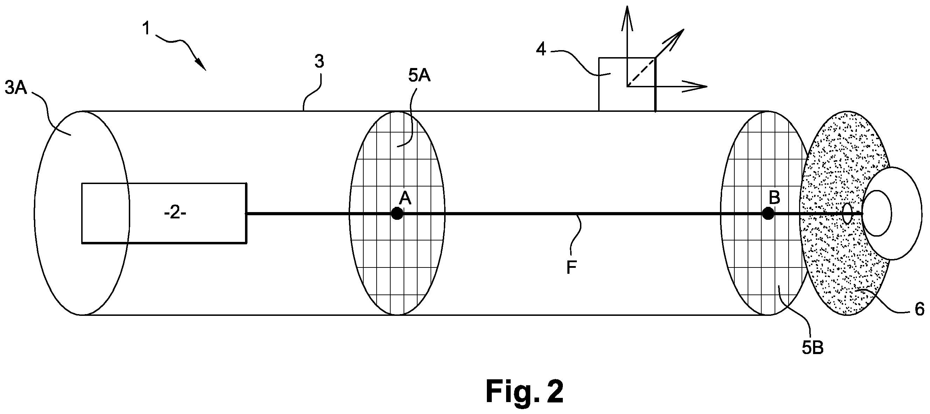

Avantageusement, le dispositif de la figure 1 peut être complété par un écran 6 troué disposé devant l'oeil de la personne et fixe par rapport à la tête par fixation sur une monture de lunette ou un casque, comme illustré sur la variante de la figure 2.

Ces éléments peuvent être réglables en position longitudinale sur le support 3 afin d'être adaptés à la vue de la personne.

Ce support 3 porte un premier capteur de position 4 qui par calibrage permet de connaître la direction du faisceau lumineux F par rapport à la position du capteur sur le support 3, par exemple par détection des points A et B.

Est ainsi défini un faisceau lumineux fin F permettant de repérer une direction correspondante à un axe visuel.

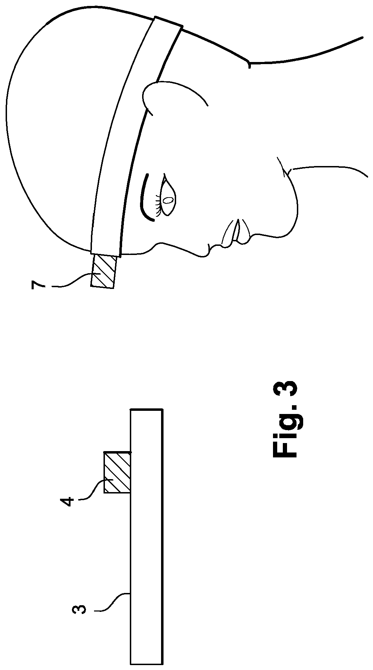

Comme schématisé sur la figure 3, le dispositif comporte un second capteur de position 7 destiné à être porté par la tête de la personne, susceptible de tourner. Fixe par rapport à la tête, par exemple solidaire d'un bandeau porté par la tête, ce capteur 7 permet de connaître la position de la tête à tout moment. A titre d'exemple, ces deux capteurs peuvent être des capteurs « Fastrak » de la société Polhemus.

Le support 3 et son faisceau lumineux F ainsi que la tête de la personne doivent être calibrés.

Ces calibrages peuvent être réalisés comme suit.

Le calibrage du support permet de calculer la cinématique permettant de passer du repère du premier capteur 4 à un repère lié à la direction de l'axe F.

Une méthode est la suivante.

L'axe F est matérialisé par un faisceau lumineux provenant de la cible 2 s'il s'agit d'une source lumineuse, soit par la cible 2 et les lames transparentes réticulées 5B et éventuellement 5A. On pointe avec un capteur mobile et ponctuel la cible 2 matérialisant l'une des extrémités de l'axe F. On pointe de la même manière l'autre extrémité de l'axe F matérialisée soit par l'intersection du faisceau avec l'autre extrémité du support, soit par la lame 5B. On peut avantageusement pointer la lame 5A en plus ou à la place de la cible 2, notamment lorsque la cible 2 est une source lumineuse dont la position est plus difficilement repérable. Un système électronique fournit les coordonnées de ces deux points, exprimé dans un repère particulier. En même temps, le système fournit les axes du repère lié au premier capteur 4 solidaire du support. Le support 3 n'a pas besoin d'être fixe pendant les mesures puisque l'on mesure à tout moment la différence de position entre le capteur 4 et le capteur mobile et ponctuel.

Un calcul simple permet de passer du repère du premier capteur 4 à la direction de l'axe F.

Le calibrage de la tête permet de calculer la cinématique permettant de passer du repère lié au second capteur 7 de la tête à un repère particulier et connu de celle-ci.

Une méthode est la suivante.

On pointe avec un capteur mobile et ponctuel le point particulier à repérer. Un système électronique fournit les coordonnées de ce point, exprimées dans un repère particulier. En même temps, le système fournit les axes du repère lié au second capteur 7 solidaire de la tête. La tête n'a pas besoin d'être fixe pendant les mesures puisque l'on mesure à tout moment la différence de position entre le capteur 7 et le capteur mobile et ponctuel.

Le capteur mobile et ponctuel précédemment utilisé pour le calibrage peut être un capteur appelé « Stylus » de la société Polhemus.

Les points particuliers et les axes nécessaires peuvent avantageusement se composer de :
la racine du nez : l'origine du repère
l'axe qui joint le centre de la pupille droite et le centre de la pupille gauche : 1^{er} axe
l'axe vertical : 2^{ème} axe
le 3^{ème} axe est calculé pour former un trièdre direct.

Le point particulier peut également avantageusement être le centre de la face arrière du verre de façon à déduire directement de la mesure de la position du CRO la distance verre- CRO.

Une fois ces points et ces axes repérés sur la personne, un calcul simple fournit un changement de repère permettant de passer, à tout moment, du repère lié au second capteur 7 au repère lié à la tête.

Une fois les calibrages effectués, le dispositif peut être utilisé comme suit.

La personne enlève ses lunettes et saisit le support 3. La personne positionne ce support devant son oeil, et bouge celui-ci jusqu'à voir la cible 2 et les cibles complémentaires éventuelles formées des lames ou écrans 5A et 5B alignées. A ce moment, l'axe de son oeil est superposé à la direction de l'axe F. Les capteurs 4 et 7, ainsi qu'un logiciel associé permettent, à ce moment, d'enregistrer la direction de l'axe visuel, ainsi que la position de sa tête. Par logiciel, on calcule ensuite la direction de l'axe visuel dans le repère lié à la tête.

On demande à la personne de bouger le tube en rotation, dans une direction quelconque. La personne bouge ensuite son oeil, et sa tête si elle le souhaite, pour aligner de nouveau les cibles ou visualiser la source lumineuse.

Une autre direction de l'axe visuel, ainsi que la position de la tête sont enregistrées.

Pour une précision minimale, cette opération est à effectuer deux fois afin de permettre la détermination de deux axes visuels.

La précision de positionnement du CRO dépend, entre autre, du nombre de directions de l'espace mesurées, ainsi que de l'écart angulaire entre les différentes droites.

A titre d'exemple, afin de garder une manipulation confortable pour la personne, on peut traiter environ 10 acquisitions de données, séparées de 20° dans toutes les directions.

On peut par exemple utiliser les neuf directions suivantes :
1/ regard droit devant
2/ vers le haut (environ 20°)
3/ vers le haut (environ 40°)
4/ vers le bas (environ -20°)
5/ vers le bas (environ - 40°)
6/ vers la droite (environ 20°)
7/ vers la droite (environ 40°)
8/ vers la gauche (environ -20°)
9/ vers la gauche (environ -40°).

On peut également utiliser neuf autres directions suivantes :
1/ regard droit devant
2/ vers le haut (environ 40°)
3/ vers le bas (environ - 40°)
4/ vers la droite (environ 40°)
5/ vers la gauche (environ - 40°)
6/ vers le haut (environ 40°) et à droite (environ 40°)
7/ vers le haut (environ 40°) et à gauche (environ - 40°)
8/ vers le bas (environ - 40°) et à droite (environ 40°)
9/ vers le bas (environ - 40°) et à gauche (environ - 40°).

Il est avantageux que les axes visuels repérés soient approximativement symétriques par rapport au regard droit devant.

Une fois la collecte des directions de l'axe visuel et des positions de la tête effectuée, la manipulation est terminée.

Le logiciel calcule, pour toutes les acquisitions, la direction de l'axe visuel dans le repère liée à la tête.

Par un algorithme approprié, le logiciel calcule alors la meilleure position du CRO à partir de toutes ces directions.

Selon une première variante de réalisation, la définition du point optimal de croisement consiste en la définition d'une surface perpendiculaire à chacun desdits axes visuels et en la définition du meilleur foyer de cette surface, ce meilleur foyer étant défini comme le centre de rotation de l'oeil.

Selon une deuxième variante de réalisation, la définition du point optimal de croisement consiste en la définition, pour chaque couple d'axes visuels, du point équidistant à ces deux axes et de distance minimale à ces deux axes, en la définition du barycentre de ces dits points, ce barycentre étant défini comme le centre de rotation de l'oeil.

Selon une troisième variante de réalisation, la définition du point optimal de croisement consiste en la définition, pour chaque couple d'axes visuels, du point équidistant à ces deux axes et de distance minimale à ces deux axes, en la définition d'une sphère de rayon minimal contenant lesdits points, le centre de cette sphère étant défini comme le centre de rotation de l'oeil.

Le dispositif conforme à l'invention peut être utilisé de différentes façons pour acquérir les droites nécessaires à la détermination du centre de rotation de l'oeil, le mode d'utilisation décrit ci-dessus n'étant qu'un exemple.

Selon un premier mode d'utilisation, la personne est libre sur sa posture générale et tient le tube dans une main.

L'avantage de ce premier mode d'utilisation est le fait que la personne est libre de ses mouvements de tête et d'oeil et conserve des positions naturelles. L'expérimentateur doit malgré tout surveiller que la personne change bien sa direction de regard. Ceci permet de respecter ses habitudes de mouvements de tête et d'oeil.

Selon un second mode d'utilisation, on place devant les yeux de la personne un écran 6 troué en certains points, dans le but de forcer la personne à regarder dans certaines directions. Dans ce cas, le dispositif conforme à l'invention comprend également cet écran disposé devant l'oeil de la personne et pourvu de zones ponctuelles translucides correspondant aux dites directions.

L'avantage de ce second mode d'utilisation est de s'assurer que les directions de regard sont bien réparties dans l'espace, ce qui permet de bien contrôler la manipulation, et de calculer la répartition des directions de regard pour obtenir la meilleure précision. Un autre avantage de ce mode d'utilisation est que l'on s'affranchit de la taille de la pupille du sujet, qui peut limiter la précision sur le positionnement de la direction de regard, en utilisant des trous de la taille souhaitée, par exemple d'un diamètre compris entre 0,5 et 2 mm.

Selon un troisième mode d'utilisation, le tube est fixé sur un banc de mesure, ce qui donne une meilleure précision sur l'alignement. On peut également utiliser un écran troué dans ce cas.

L'avantage de ce troisième mode d'utilisation est de permettre une meilleure précision et stabilité de l'alignement en évitant les petits tremblements liés à la manipulation du tube.

Il est également possible de déterminer, pour un même oeil, plusieurs positions du CRO correspondantes à plusieurs couples d'axes visuels. La position du CRO est alors fonction d'un couple d'axes visuels.

Par exemple, le regard droit devant est pris comme axe de référence et plusieurs axes visuels symétriques de part et d'autre du regard droit devant sont déterminés. A chaque couple (regard droit devant, autre axe visuel) est déterminée une position du CRO.

Un tel ensemble de position du CRO peut être exploité dans le domaine ophtalmique.

## Revendications

1. Procédé de détermination du centre de rotation d'un oeil (CRO) d'une personne par rapport à un repère lié à la personne ou à sa paire de lunettes, consistant en la détermination de l'axe visuel de cette personne dans au moins deux directions non parallèles au moyen d'une cible (2) visualisée et la définition d'un point optimal dit de croisement de ces axes comme centre de rotation de l'oeil, **caractérisé en ce qu'**au moins deux positions relatives de cette cible (2) et de la tête de la personne sont mesurées, et **en ce qu'**il consiste, pour chacune de ces directions non parallèles, à saisir, positionner et bouger ladite cible (2) constituée d'une source de lumière ponctuelle disposée à une extrémité (3A) d'un support tubulaire (3) constitué uniquement d'un tube et portant un premier capteur de position (4), calibré pour connaître la direction du faisceau lumineux de ladite lumière ponctuelle, avec son autre extrémité (3B) en face de l'oeil de la personne, de façon à superposer l'axe visuel avec chaque direction du faisceau lumineux de la source de lumière, un second capteur de position (7) étant placé sur la tête de la personne et un logiciel associé aux deux dits capteurs assurant l'enregistrement de la direction de l'axe visuel, ainsi que la position de la tête correspondante, puis le calcul de l'axe visuel dans un repère lié à la tête.

2. Procédé selon la revendication précédente, **caractérisé en ce qu'**il comporte également une deuxième cible transparente destinée à être disposée en face de l'oeil de la personne.

3. Procédé selon l'une des revendications précédentes, **caractérisé en ce que** ledit support est un tube de faible diamètre.

4. Procédé selon l'une des revendications précédentes, **caractérisé en ce qu'**il comporte également une autre cible transparente destinée à être disposée entre ladite source lumineuse et le milieu du support.

5. Procédé selon l'une des revendications précédentes, **caractérisé en ce qu'**il comprend un écran fixe par rapport à la tête de la personne et disposé devant l'oeil de la personne et pourvu de zones ponctuelles translucides correspondantes aux dites directions.

6. Procédé de détermination du centre de rotation selon l'une des revendications précédentes, au moins deux positions relatives de ladite cible (2) et de la tête de la personne étant mesurées **caractérisé en ce que** la définition du point optimal de croisement consiste en la définition, pour chaque couple d'axes définis par lesdites directions de l'axe visuel, du point équidistant à ces deux axes et de distance minimale à ces deux axes, en la définition du barycentre de ces dits points, ce barycentre étant défini comme le centre de rotation de l'oeil.

7. Procédé de détermination du centre de rotation selon l'une des revendications précédentes, au moins deux positions relatives de ladite cible (2) et de la tête de la personne étant mesurées **caractérisé en ce que** la définition du point optimal de croisement consiste en la définition, pour chaque couple d'axes définis par lesdites directions de l'axe visuel, du point équidistant à ces deux axes et de distance minimale à ces deux axes, en la définition d'une sphère de rayon minimal contenant lesdits points, le centre de cette sphère étant défini comme le centre de rotation de l'oeil.

8. Procédé selon la revendication 6 ou 7, **caractérisé en ce que** lesdites directions repérées sont sensiblement symétriques par rapport au regard droit devant de l'oeil.

## Patentansprüche

1. Verfahren zur Bestimmung des Rotationszentrums eines Auges (CRO) einer Person in Bezug auf einen Bezugspunkt, der mit der Person oder ihrer Brille verbunden ist, das in dem Bestimmen der Sehachse dieser Person in mindestens zwei nicht parallelen Richtungen mittels eines visualisierten Ziels (2) und dem Definieren eines sogenannten optimalen Schnittpunktes dieser Achsen als das Rotationszentrum des Auges besteht, **dadurch gekennzeichnet, dass** mindestens zwei relative Positionen dieses Ziels (2) und des Kopfes der Person gemessen werden, und dass es darin besteht, für jede dieser nicht parallelen Richtungen, das Ziel (2) zu erfassen, zu positionieren und zu bewegen, das aus einer Punktlichtquelle gebildet ist, die an einem Ende (3A) eines rohrförmigen Trägers (3), der nur aus einem Rohr gebildet ist und einen ersten Positionssensor (4), der kalibriert ist, um die Richtung des Lichtstrahls des Punktlichts zu erkennen, mit ihrem anderen Ende (3B) gegenüber von dem Auge der Person derart angeordnet ist, dass die Sehachse mit jeder Richtung des Lichtstrahls der Lichtquelle überlagert wird, einen zweiten Positionssensor (7), der auf dem Kopf der Person angeordnet ist, und eine Software trägt, die mit den zwei Sensoren verbunden ist, die das Aufzeichnen der Richtung der Sehachse, der Position des entsprechenden Kopfes und dann das Berechnen der Sehachse in einem Bezugspunkt, der mit dem Kopf verbunden ist, gewährleistet.

2. Verfahren nach dem vorhergehenden Anspruch, **dadurch gekennzeichnet, dass** es auch ein zweites durchsichtiges Ziel aufweist, das dazu bestimmt ist, gegenüber von dem Auge der Person angeordnet zu werden.

3. Verfahren nach einem der vorhergehenden Ansprüche, **dadurch gekennzeichnet, dass** der Träger ein Rohr mit geringem Durchmesser ist.

4. Verfahren nach einem der vorhergehenden Ansprüche, **dadurch gekennzeichnet, dass** es auch ein anderes durchsichtiges Ziel aufweist, das dazu bestimmt ist, zwischen der Lichtquelle und der Mitte des Trägers angeordnet zu werden.

5. Verfahren nach einem der vorhergehenden Ansprüche, **dadurch gekennzeichnet, dass** es einen Bildschirm aufweist, der fest bezüglich des Kopfes der Person ist und vor dem Auge der Person angeordnet ist und mit lichtdurchlässigen, punktförmigen Bereichen versehen ist, die den Richtungen entsprechen.

6. Verfahren zur Bestimmung des Rotationszentrums nach einem der vorhergehenden Ansprüche, wobei mindestens zwei relative Positionen des Ziels (2) und des Kopfes der Person gemessen werden, **dadurch gekennzeichnet, dass** die Definition des optimalen Schnittpunktes in der Definition für jedes Paar von Achsen, die durch die Richtungen der Sehachse definiert werden, des Punktes, der von diesen zwei Achsen äquidistant und mit einem Mindestabstand von diesen zwei Achsen ist, in der Definition des Schwerpunktes dieser Punkte besteht, wobei dieser Schwerpunkt als das Rotationszentrum des Auges definiert wird.

7. Verfahren zur Bestimmung des Rotationszentrums nach einem der vorhergehenden Ansprüche, wobei mindestens zwei relative Positionen des Ziels (2) und des Kopfes der Person gemessen werden, **dadurch gekennzeichnet, dass** die Definition des optimalen Schnittpunktes in der Definition für jedes Paar von Achsen, die durch die Richtungen der Sehachse definiert werden, des Punktes, der von diesen zwei Achsen äquidistant und mit einem Mindestabstand von diesen zwei Achsen ist, in der Definition eines Bereichs des Mindestradius, der diese Punkte enthält, besteht, wobei die Mitte dieses Bereichs als das Rotationszentrum des Auges definiert wird.

8. Verfahren nach Anspruch 6 oder 7, **dadurch gekennzeichnet, dass** die erfassten Richtungen im Wesentlichen symmetrisch in Bezug auf den Blick geradeaus vor dem Auge sind.

## Claims

1. Method for determining the rotation centre of an eye (ERC) of a person with respect to a coordinate system associated with the person or with his/her pair of spectacles, consisting in determining the visual axis of this person in at least two nonparallel directions by means of a viewed target (2) and defining an optimal point that is known as the optimal crossover point of these axes as the eye rotation centre, **characterized in that** at least two relative positions of this target (2) and of the head of the person are measured, and **in that** the method consists, for each of these nonparallel directions, in seizing, positioning and moving said target (2), which consists of a point light source placed at one end (3A) of a tubular holder (3) that consists merely of a tube and that bears a first position sensor (4) that is calibrated to know the direction of the light beam of said point light, with its other end (3B) facing the eye of the person, so as to superpose the visual axis with each direction of the light beam of the light source, a second position sensor (7) being placed on the head of the person and a software package associated with said two sensors recording the direction of the visual axis and the corresponding position of the head, then calculating the visual axis in a coordinate system associated with the head.

2. Method according to the preceding claim, **characterized in that** it also includes a transparent second target intended to be placed facing the eye of the person.

3. Method according to one of the preceding claims, **characterized in that** said holder is a tube of small diameter.

4. Method according to one of the preceding claims, **characterized in that** it also includes another transparent target intended to be placed between said light source and the middle of the holder.

5. Method according to one of the preceding claims, **characterized in that** it comprises a screen that is fixed with respect to the head of the person and placed in front of the eye of the person and provided with translucent point-like zones corresponding to said directions.

6. Method for determining the rotation centre according to one of the preceding claims, at least two relative positions of said target (2) and of the head of the person being measured, **characterized in that** the definition of the optimal crossover point consists in defining, for each pair of axes defined by said directions of the visual axis, the point equidistant from these two axes and of minimum distance from these two axes, in defining the centroid of these said points, this centroid being defined as the eye rotation centre.

7. Method for determining the rotation centre according to one of the preceding claims, at least two relative positions of said target (2) and of the head of the person being measured, **characterized in that** the definition of the optimal crossover point consists in defining, for each pair of axes defined by said directions of the visual axis, the point equidistant from these two axes and at a minimum distance from these two axes, in defining a sphere of minimum radius containing said points, the centre of this sphere being defined as the eye rotation centre.

8. Method according to Claim 6 or 7, **characterized in that** said located directions are substantially symmetrical with respect to the straightahead gaze of the eye.
